# EUROPEAN PATENT APPLICATION

(11) **EP 3 372 282 A1**
(43) Date of publication of application: **12.09.2018**
(21) Application number: 18159881.4
(22) Date of filing: 05.03.2018
(51) Int. Cl.: A61Q 13/00, A61K 8/06, A61K 8/34, A61K 8/37, A61K 8/86, A61K 8/44

(54) **ETHANOL-FREE, AQUEOUS MICROEMULSION PERFUME COMPOSITIONS**

(30) Priority: 08.03.2017 US 201762468739 P; 01.03.2018 US 201815909143
(71) Applicant: Solinas, Sylvain, Egham TW20 0RR (GB); Schmeling, Marianne, 41352 Korschenbroich (DE); Hoffmann, Wundriari, Camberley, Surrey GU16 9RF (GB)
(72) Inventor: Solinas, Sylvain, Egham TW20 0RR (GB); Schmeling, Marianne, 41352 Korschenbroich (DE); Hoffmann, Wundriari, Camberley, Surrey GU16 9RF (GB)
(74) Representative: Hanna Moore + Curley

(57) **Abstract**

Compositions based upon an ethanol-free aqueous microemulsion having an aqueous phase including at least a preservative system, a discontinuous perfume oil phase and a surfactant system are disclosed. The compositions are stable microemulsions, resist microbial growth and can be used as perfume components in almost all perfume products.

## Description

### TECHNICAL FIELD

The invention relates to a perfume composition that is an ethanol-free microemulsion of a surfactant system, a preservative system, fragrance oil and water. The surfactant system comprises a primary surfactant and a co-surfactant. The surfactant system enables formulation and stabilization of the microemulsion of the fragrance oil in a continuous phase of water. The preservative system maintains the composition free from microbial growth for an extended period.

### BACKGROUND OF THE INVENTION

The present invention relates to an at least substantially transparent, ethanol-free perfume which is formulated as an aqueous microemulsion composition. Embodiments of the microemulsion composition, include fragrances, Eaux de Toilettes, body sprays, body deodorants, refreshing and cleaning wet towels, aqueous cosmetic compositions, household cleaners, and air fresheners.

Since the time of Cleopatra, the perfume industry has produced fragrant, attractive oils and emollients that incorporate ethanol as a base. The ethanol enables aqueous dissolution of the oily ingredients such as perfume. In addition to ethanol as an aqueous solubilizer, perfumers often use other more lipophilic alcohols such as butanol. However, higher weight alcohols can deliver malodorous and off-scents to the perfume compositions. Lower weight alcohols such as ethanol based perfumes tend to dry the skin and bear a societal stigma owing to their intoxicating character. Such ethanol-containing perfumes and fragrant topical compositions are not acceptable for various reasons such as skin sensitivity, infant safety, and religious prohibition of use of ethanol for some consumers.

Another problem specific to the perfume industry is that of the typical presence of alcohol. Ethanol is very widely used as a solvent in the preparation of perfumed compositions such as perfumes, Eaux de toilette, Eaux de Cologne, or deodorizing compositions for example. Ethanol enables good solubilization of the perfuming ingredients available to the perfumer. It thus constitutes the principle vector used in perfumed body-care products (perfumes, Eaux de toilette, deodorants, after-shaves etc.) and, as it is virtually odorless, it is a very good solvent which evaporates rapidly thus imparting a cooling sensation. For these reasons, the majority of commercially available perfuming compositions contain ethanol, generally in a proportion of 50 to 95% by volume.

Development of ethanol-free aqueous perfume compositions can provide societal benefits. Aqueous compositions have such benefits as the environmentally friendly character of water, non-flammable compositions and ethnic and societal acceptance. The perfume industry accordingly has attempted to develop ethanol-free compositions.

Nevertheless, ethanol-free aqueous perfume compositions have their own problems with formulation and consumer acceptability. Aqueous based perfume compositions are translucent to opaque in appearance, tend to promote oxidative degradation of the odoriferous components and leave sticky residues that can be long lasting. The organic solubilizing agents not only contribute to these undesirable characteristics but also deliver malodor notes and skin hypersensitivity.

The replacement of ethanol is very challenging for the perfumer due to the beneficial characteristics of this ingredient in perfume formulations. Indeed, ethanol is an ideal solvent for perfumery ingredients, e.g. it evaporates rapidly and does therefore not disturb the odor of the perfume after its short time of evaporation. Another key characteristic of ethanol is its wetting power, which allows good spreadability of the perfume on the skin and in turn controls the diffusion of the perfume at a later step. The fast evaporation of ethanol also adds a somewhat refreshing aspect to the formulation; a characteristic that is highly appreciated by the customer.

The immiscible relation of water and typical perfume oils lends itself to the well-known phenomenon of oil in water emulsions. Oil-in-water emulsions are well known in the cosmetics industry and in dermatology, in particular for the preparation of cosmetic products such as creams, lotions, tonics or serums. However, these emulsions always contain a small proportion of a low-molecular-weight alcohol.

The problems associated with the formulation of emulsions in the dermatology and cosmetics industry are not the same as those encountered in perfumery. The objective of hair-care and skin-care products and of products for cleaning the hair and skin is to optimize the penetration of active substances into the superficial layers of the skin. Moreover, these cosmetic emulsions are of a highly specific composition and are characterized in particular by the fact that their oil phase comprises a large variety of active substances such as natural or synthetic oils, hydrocarbons, halogenated hydrocarbons, mineral acid esters or silicones, differing according to the desired application. In contrast with a cosmetic composition, a perfuming composition is a perfume carrier the primary function of which is to impart a scent to a product. In the form of an emulsion, its oil phase is made up essentially of perfuming ingredients. It is clear that the problems to be resolved when formulating emulsions, such as in particular optimization of the parameters capable of influencing the stability of the product, are differ for different industries.

Apart from exhibiting chemical stability, an emulsion must also meet certain requirements regarding physical stability. For example, one of the phenomena typically associated with the physical instability of an emulsion is the ascending and descending movement of the dispersed droplets relative to the continuous phase. These phenomena are called creaming or sedimentation, respectively.

An additional complication focuses on the characteristics and differences between microemulsions and emulsions. Microemulsions and emulsions constitute two distinctly different kinds of dispersed systems. Whereas emulsions such as salad oil in water are unstable systems, microemulsions are stable and form spontaneously when oil, water, surfactants and co-surfactants are mixed together. The thermodynamic stability of a microemulsion is revealed in particular by the fact that, in contrast with an emulsion, the mean droplet size in the system does not vary over time. The two disperse systems also differ in respect of their optical properties.

While the foundational understanding of microemulsions seems to suggest microemulsions as formulations for aqueous based perfumes, it is also recognized that oil in water emulsions typically incorporate significant quantities of surfactants relative to the oil phase. The need for such large quantities of surfactants in oil in water emulsions will considerably limit the proportion of perfume that can be added to the mixture. In addition, many surfactants deliver olfactory responses that may mask, change or otherwise affect the olfactory response to the perfume component. This is why a disperse system of the microemulsion kind will typically be unsuitable as a non-ethanol perfume. Furthermore, microbial growth tends to be problematic even though typical preservatives can be added.

Therefore, there is a need to develop an ethanol-free aqueous cosmetic composition approved for cosmetic uses that provides an efficient, optically clear, non-greasy, non-tacky fragrance microemulsion without untoward overtones of scent and that resist microbial growth.

### SUMMARY OF THE INVENTION

The present invention is directed to a composition of an ethanol-free aqueous microemulsion of a water immiscible fragrance oil that exhibits extended shelf life stability, resistance toward microbial and yeast growth, and incorporates a surfactant system at concentrations and with components that do not significantly mask, alter, adulterate or otherwise change the olfactory properties of the fragrance oil. In addition, the microemulsion composition embodiments of the invention resist microbial growth for significantly long periods of time.

The composition is an aqueous microemulsion of a surfactant system, a preservative system, fragrance oil and water. The composition is free of ethanol. The microemulsion is a construct of micelles of lipophilic components suspended in a continuous aqueous phase. The micelles neither coagulate nor settle to form a separate continuous phase of the lipophilic components. The separation of the micelles as a microemulsion is maintained at least in part by the size of the micelles ranging from about 10 to about 100 nanometers, preferably about 20 nanometers to about 50 nanometers in micelle cross section. The separation of the micelles as a microemulsion is also maintained in part by the surfactant system. The aqueous microemulsion composition of the invention can maintain a stable shelf life of at least about three years. The stability is measured by the substantial freedom from coagulation or settlement of the micelle discontinuous phase and by the clarity of the composition.

The surfactant system of the composition includes at least a primary surfactant and a co-surfactant/hydrotrope. The primary surfactant exhibits solubility in aqueous and lipophilic media due to its dual character of lipophilicity and hydrophilicity. The dual character eliminates surface tension, provides micelle repulsion and eliminates phase separation. The co-surfactant/hydrotrope is not a surfactant. It functions as a micellar promotor in that among other functions it enhances the dual character of the primary surfactant so as to enable use of lower concentrations of the primary surfactant. A semi-polar character coupled with its low or negligible volatilization and moderate to low lipophilic activity characterize the co-surfactant/hydrotrope. The surfactant system also is cosmetically and topically acceptable in that it does not cause substantial skin irritation, dermal roughing or other dermal malconditions.

The primary surfactant has a nonionic form with having a poly(alkyl diol) tail and a head of a fatty acid, fatty alcohol, saccharide or polysaccharide, saccharide alcohol such as sorbitol or sorbitan or a saturated or unsaturated, mono, di or triglyceride optionally containing hydroxyl groups. A single nonionic surfactant or a combination of nonionic surfactants may be formulated as the primary surfactant. The poly(alkyl diol) tail of the embodiments of the primary surfactant may be a polyethylene glycol, polypropylene glycol, polybutylene glycol, or other polymer of a C2 to C8 diol having ether linkages of the alkyl groups and hydroxyl terminating the polymer (hereinafter a polyglycol). The embodiments of the head include hydrophobic moieties that contain groups such as hydroxyls and/or carboxyls that can be covalently bonded to the polyglycol. These embodiments include moieties such as but not limited to fatty acid, fatty alcohol, saccharides and derivatives, polysaccharides and derivatives, saccharide alcohols and glycerides. For the glyceride embodiment, the glyceride is a mono, di or tri ester having three, four or five carbons for which one, two or three of the hydroxyls is (are) esterified with a saturated or unsaturated fatty acid having 8 to 30 carbons. The fatty acid moiety may optionally contain one or two hydroxyl groups along the hydrocarbon chain. One or more polyglycols may be joined to one or more hydroxyls of the glyceride, or may be joined to one or more hydroxyls of the fatty acid moiety if the fatty acid moiety contains a hydroxyl, or may be a combination of polyglycols joined to the hydroxyls of the fatty acid moiety and the glyceride. If polyglycol(s) is (are) joined to one or two hydroxyls of the glyceride, the glyceride will be mono or diglyceride, i.e., a mono or di ester with the fatty acid.

The co-surfactant/hydrotrope is a C4 to C15 alkyl diol or triol or a combination of two or more diols or triols. Preferably the co-surfactant/hydrotrope is a C5 to C12 alkyl diol or triol or a combination of two or more diols or triols. Diols are especially preferred.

The preservative system has anti-oxidative, anti-microbial, anti-fungal properties so that the perfume oil is substantially protected against degradative oxidation, microbial attack and fungal growth. The preservative system is also cosmetically and topically acceptable in that it does not cause substantial skin irritation, dermal roughing or other dermal malconditions. Nevertheless, the preservative system protects the microemulsion composition embodiments of the invention from microbial growth for significant periods of time.

The preservative system includes at least o-cymen-5-ol (3-methyl-4-isopropyl phenol) and EDTA in combination with antibacterial and/or antimicrobial compounds such as short chain alkanoic acids, aromatic acids and alcohols, terpenoids, and similar antimicrobials. Specific examples of antimicrobial compounds include phenoxyethanol and a mixture ofparabens. The parabens mixture includes methyl, ethyl, propyl and butyl parabens. O-cymen-5-ol is a positional isomer of thymol, 5-methyl-2-isopropyl phenol. Both have antibacterial and antimicrobial activity and either can be employed in the preservative system. However, thymol is the aromatic constituent of the herb thyme and has a pleasant but distinctive fragrance which may lend an additional note to certain fragrance oils used in the compositions of the invention. O-cymen-5-ol does not have a distinctive fragrance of its own. It is therefore preferred as an antibacterial and antimicrobial constituent of the preservative system.

The fragrance oil is lipophilic, substantially aqueous immiscible and delivers fragrant scents spanning a wide range of olfactive responses. The fragrance oil may be any one or combination of aromatic extracts, extracts of odorous animal or insect sacs, extracts of plant materials and synthetic odorants including but not limited to esters. The fragrance oil is the primary component of the micelles of the microemulsion. Other lipophilic components such as members of the preservative system may also be present in the fragrance oil micelles. Fragrance and perfume oils are further explained in the following definitions section.

The present invention is further directed to a method for producing the ethanol-free microemulsion of the invention as well as to the use of the composition as perfume by application of the composition to the skin of a human.

Specific embodiments of the invention include an aqueous microemulsion composition composed of a perfume oil micelle phase having a surfactant system composed of a primary surfactant or surfactants including a fatty acid moiety having from 15 to 50 carbons esterified with a polyol moiety such as PEG or PPG having from 6 to 80 monomeric units as well as the fatty alcohol moiety corresponding to the fatty acid moiety ethoxylated with the foregoing polyol moiety. The fatty acid moiety may be a saturated of unsaturated form and may additionally optionally hydroxylated. Included as examples of unsaturated fatty acids are oleic, elaidic, vaccenic, linoleic, arachidonic, eicosapentenoic, erucic, docosahexenoic and castor oil precursor acids. Examples of saturated fatty acids are hydrogenated forms of the foregoing unsaturated fatty acids as well as capric, lauric, myristic, palmitic, stearic, arachidic, behenic, lignoceric and cerotic acids. Additional examples include the mono, di and tri hydroxylated forms of these saturated and unsaturated fatty acids. Especially preferred fatty acid moieties are hydrogenated castor oil, hydroxylated, hydrogenated castor oil, hydrogenated arachidonic acid and a hydroxylated form thereof, behenic acid and a hydroxylated form thereof and lignoceric or cerotic acid and hydroxylated forms therof. Especially preferred fatty alcohol moieties are the fatty alcohols corresponding to the especially preferred fatty acid moieties as well as the hydroxylated forms thereof. More especially preferred fatty acid moieties are hydrogenated, hydroxylated castor oil, hydrogenated, hydroxylated arachidonic acid, hydrogenated, hydroxylated behenic acid and hydrogenated, hydroxylated lignoceric acid as well as the corresponding fatty alcohol forms thereof. Most especially preferred fatty acid moieties are hydrogenated, hydroxylated castor oil and hydrogenated, hydroxylated behenic acid and the corresponding fatty alcohol forms thereof. Specifically, most preferred moieties include hydrogenated castor oil esterified with PEG 40 and the fatty alcohol buteth 26 ethoxylated with PPG 26 and a combination thereof.

Specific embodiments of the co-surfactant component of the surfactant system include alkane diols of 4 to 15 carbons wherein the hydroxyl groups are positioned on the alkane chain other than at a vicinal position (i.e., a 1,2 diol arrangement). Preferred diols include pentane diol, hexane diol, heptane diol, octane diol and nonane diol. Especially preferred diols include 1, 5-pentane diol and 1,6-hexane diol. More especially preferred diols include 1,5-pentane diol.

Specific embodiments of the preservative system include combinations of any one or more of EDTA as a chelator of metal ions, o-cymen-5-ol, phenoxy ethanol and parabens. Especially preferred is a combination of EDTA, o-cymen-5-ol, phenoxyethanol and methyl, ethyl, propyl and butyl parabens.

### DETAILED DESCRIPTION OF THE INVENTION

The present invention is directed to an ethanol-free composition of one or more perfume oils microemulsified in a continuous aqueous medium of water, a surfactant system and a preservative system. The composition is a stable microemulsion with concentrations of the surfactant system and preservative system that do not alter, adulterate, modify or otherwise change the olfactive sensory response of humans to the perfume oil. Typical microemulsions require concentrations of surfactant that is at least equal to or exceeds the concentration of the dispersed phase, such as the perfume micelles. The inventive microemulsion utilizes a surfactant system comprising a primary surfactant and a co-surfactant/hydrotrope to achieve this result. This system significantly lowers the amount of primary surfactant needed for effective microemulsification and stabilization.

### Definitions

Unless defined otherwise, all technical and scientific terms used herein have the same meaning as commonly understood by a person of ordinary skill in the art.

As used in the specification and the appended claims, the singular forms "a," "an" and "the" include plural referents unless the context clearly dictates otherwise.

The term "may" in the context of this application means "is permitted to" or "is able to" and is a synonym for the term "can." The term "may" as used herein does not mean possibility or chance.

The term "about" is understood to mean ±10 percent of the recited number, numbers or range of numbers.

The term "about 0 wt%" is understood to mean that no substance, compound or material to which zero (0) refers is present, up to a negligible but detectable amount is present, assuming that the detectability can be determined on a parts-per-million basis.

The molecular weight of a polymer or oligomer used according to the invention may be measured by a weight average molecular weight. The distribution of molecules of different molecular weights of a polymer or oligomer used according to the invention is determined by its polydispersity index. Molecular weight is expressed as daltons (Da) and kiloDaltons (kDa). The acronym "wmw" stands for weight average molecular weight. Polydispersity is a unit-less number and indicates the breadth of the Gaussian curve plotted as the molecular weight of individual molecules (X axis) against the number of molecules at each molecular weight (Y-axis).

Where features or aspects of the invention are described in terms of Markush groups, those skilled in the art will recognize that the invention is also thereby described in terms of any individual member or subgroup of members of the Markush group. For example, if X is described as selected from the group consisting of methyl, ethyl or propyl, claims for X being methyl and claims for X being methyl and ethyl are fully described. Moreover, where features or aspects of the invention are described in terms of Markush groups, those skilled in the art will recognize that the invention is also thereby described in terms of any combination of individual members or subgroups of members of Markush groups. Thus, for example, if X is described as selected from the group consisting of bromine, chlorine, and iodine, and Y is described as selected from the group consisting of methyl, ethyl, and propyl, claims for X being bromine and Y being methyl are fully described.

If a value of a variable that is necessarily an integer, e.g., the number of carbon atoms in an alkyl group or the number of substituents on a ring, is described as a range, e.g., 0-4, what is meant is that the value can be any integer between 0 and 4 inclusive, i.e., 0, 1, 2, 3, or 4.

"Ethanol-free" as used herein means essentially free of ethyl alcohol. "Essentially free" in this context means ethanol is present at less than 0.1 wt %, preferably less than 0.01 wt %, and more preferably 0.00 wt % relative to the complete composition. While an aspect of the invention is achievement of an essentially ethanol-free perfume, some commercially available starting materials for embodiments of the invention may contain ethanol. This ethanol is removed by evaporation or other means before formulation of such starting materials into the compositional embodiments of the invention. Nevertheless, a small amount of ethanol such as no more than about 1 wt% may remain under certain circumstances. This minor amount of ethanol may be present without negating the social and/or physical characteristics of the compositional embodiments of the invention. Such minor amounts would mimic the ethanol concentrations in, for example, citrus fruits such as orange, grapefruit and clementine. For example, Davis in "Florida State Horticultural Society Journal", 1970 at pages 294 et seq., reported that oranges contain as much as 0.5 wt% ethanol at maturity. Thus, compositional embodiments of the invention with a minor amount of ethanol such as less than or about 1 wt%, preferably less than 0.5 wt%, more preferably less than or about 0.1 wt% would present an amount of ethanol like that naturally present in citrus fruits. Most preferable, however, are compositional embodiments of the invention that are completely free of any trace of ethanol.

A "microemulsion" as the term is used herein denotes a transparent mixture of (i) two immiscible liquids, and (ii) at least one amphiphile (exemplified by surfactants). One of the liquids is a continuous phase which is the aqueous phase of the microemulsion embodiments of the invention while the other liquid is the discontinuous phase which constitutes micelles primarily consisting of the perfume oil of the microemulsion embodiments of the invention. Microemulsions are transparent or translucent, and do not display the opalescence of standard emulsions. The size of the micelles is small enough so the resulting mixture is optically clear or translucent. Microemulsion micelle sizes are variously defined in the art with a cross sectional size typically below 0.2 nanometers, preferably 0.15 nanometers. The clarity of these compositions is advantageous in cosmetic applications. The microemulsion embodiments of the invention are thermodynamically stable.

The term "optically clear" as used herein defines a composition that is "transparent" (i.e. transmitting light without distortion) which means that the size of the particles in the composition are reduced to a size where the particles are not observable with optical (visual) means.

As used herein the weight percentages given herein are relative to the total weight of the complete and total perfumed aqueous microemulsion composition unless otherwise explicitly stated. It is also understood that water is present in the appropriate amount to reach the 100 weight percent, i.e. is added to completion to 100% w/w.

The term "fragrance oil" or "perfume oil" as used herein maintains its typical and usual meaning in the art, i.e. a lipophilic organic liquid that is essentially insoluble in water and being composed of one or more ingredients of natural and/or synthetic source that produce any kind of olfactive response including attractive, pleasant, flowery, aromatic, pungent, woody, as well as unpleasant odors. In particular, fragrance oil includes any perfuming ingredient or, as happens more often, any mixture of perfuming ingredients currently used in perfumery, e.g. of compounds capable of imparting a hedonic olfactive effect to the composition to which they are added. The perfuming ingredients can be of natural or synthetic origin. Generally, such oils belong to chemical classes as varied as alcohols, aldehydes, ketones, esters, ethers, acetates, nitriles, terpenes, hydrocarbons, nitrogenous or sulphurous heterocyclic compounds and essential oils of natural or synthetic origin. The natural and/or synthetic fragrance oils include oil soluble perfume oils, which may or may not be in a mixture with water soluble perfume oils. The oil soluble perfume oils are natural, or nature-identical essential oils, such as orange oil, pine oil, peppermint oil, eucalyptus oil, lemon oil, clove leaf oil, cedarwood oil, bergamot oil, rosemary oil, patchouli oil, lavandin oil, chamomile, jasmine oil, spike oil, rose oil, vetiver oil, fennel oil, anise oil, thyme oil, germanium oil, lavender oil, marjoram or menthol. An animal fragrance, is for example musk, castoreum, amber or zibet. Spagyric essences are also known in the art. They are made by collecting certain herbs while they are flowering, and fermenting in the presence of water and yeast, steam distilling off the active ingredients, and concentrating the distillate to the spagyric basic essence. The remaining mash is calcinated, and the basic essence and the calcinated mash are combined to give the final spagyric essence. Synthetic fragrant ingredients are for example synthetic essential oils, such as composed of single compounds, such as linalol, terpineol, nerol, citronelal, benzaldehyde, cinnamon aldehyde, vanillin or methyl acetophenone. The fragrance materials may also be synthetic oil-soluble perfume oils, selected from the usual group consisting of fragrant hydrocarbons, alcohols, ketones, aldehydes, ethers, esters, aldehydes, acetals and polyene compounds. This term also encompasses any mixture of perfume oils described above, or perfume concentrates or bases with preferably non-ethanolic diluents. The nature of these ingredients can be found in specialized books of perfumery and flavor ingredients, e.g. in S. Arctander (Perfume and Flavor Chemicals, Montclair N.J., USA 1969), or similar textbooks of reference. The selection of such ingredients is carried out by the perfumer without particular difficulty, on the basis of her/his general knowledge and as a function of the nature of the product to be modified and of the desired sensory effect, i.e. the perfuming or taste effect that is to be imparted to the consumer product to be perfumed or flavored.

The terms "fragrance oil" and "perfume oil" are interchangeable terms having the same meaning throughout this application.

As used herein, the term "hydrotrope" means a compound that is able to decrease the surfactant concentration necessary to form a microemulsion at a given oil concentration in aqueous media and/or to increase the dispersive capacity of the microemulsion. These molecules are surface active, i.e. decrease the surface tension of water but cannot replace the surfactant. As components of embodiments of the present invention, hydrotropes are co-surfactants. Use of a hydrotrope enables replacement and/or reduction of the amount of surfactant that enables formation of a transparent, clear microemulsion.

### DESCRIPTION OF MICROEMULSION COMPOSITION AND THE PREFERRED EMBODIMENTS

The ethanol-free, perfumed aqueous microemulsion compositional embodiments of the present invention include at least the following components: (A) one or more fragrance oils, (B) water, preferably de-ionized water, (C) a surfactant system of a primary surfactant and a co-surfactant/hydrotrope wherein the co-surfactant/hydrotrope is preferably a diol, and (D) a preservative system. The embodiments of the present invention may also optionally contain one or more additional ingredients, such as antioxidants, chelating agents, UV filters, preservatives, thickening agents, cosmetic active ingredients, moisturizers, humectants, emollients, opacifiers, pearly gloss impacting substances, pigments, colorants, dyes and antifoams.

The weight contributions of each component relative to the total weight of the microemulsion composition are:
1. for the fragrance oil or oils - about 4.0 wt% to about 10.0 wt%, component A;
2. for the surfactant system - about 1.0 wt% to about 25 wt%, component B;
3. for the preservative system - about 1.0 wt% to about 5 wt%, component C;
4. for water - a remainder to provide 100% by weight, component D.

The weight percentages of optional ingredients are provided in the following text. Their amounts will be added to the weight percentages of fragrance oil, surfactant system and preservative system and the sum subtracted from 100% will be the water percentage.

As described in the Summary, the composition embodiments of the invention are stable aqueous microemulsions wherein the discontinuous oil phase is composed of micelles at least of fragrance oil and the continuous aqueous phase includes the preservative system and the surfactant system. The surfactant system in one aspect forms a stabilizing interface between the aqueous phase and the micelles of the oil phase.

The aqueous microemulsion fragrance composition embodiments according to the present invention may be prepared, for example, by simple mixing of all the ingredients; for example, by hand stirring or if need be by using a mechanical mixer (i.e. by some mechanically agitating means), the components of the present invention, and any optional components, to form a homogeneous mixture. The components of the present invention may be added together into a suitable reaction vessel and mixed in any order, using conventional processes well known to those skilled in the art. The microemulsion may be produced at room temperature or at an elevated temperature, for example up to 90° C., preferably up to 55° C. can be employed.

A suitable exemplary method may be practiced by dissolving into the water the surfactant system, to form a clear micellar solution. To the resulting micellar solution are added under gentle stirring the preservative system and when included the optional ingredients to form an initial pre-microemulsion. Next, the fragrance oil and any optional water immiscible ingredients are combined to form an oil solution. Under gentle mixing, the oil solution is added slowly and preferably dropwise to the pre-microemulsion to form the microemulsion composition embodiments. The pre-microemulsion can easily disperse the oil solution to form an isotropic clear, microemulsion product. High mechanical forces such as shear forces are not necessary to manufacture the present microemulsion.

The fragrance oil component A of the microemulsion embodiments of the present invention may be any water immiscible natural or synthetic fragrance/perfume oil as defined in the foregoing definitions section.

The disperse micellar phase of the microemulsion embodiments is essentially composed of perfuming ingredients. More particularly, the disperse phase is composed of one or more fragrance oils in a proportion of about 50 to about 99% by weight, preferably about 70 wt% to about 99 wt%, more preferably about 80 wt% to about 99 wt% relative to the total weight of the disperse phase.

The disperse micellar phase is present in a quantity varying between about 2.0 wt% and about 15.0 wt% relative to the total weight of the complete microemulsion, preferably between about 2.0 wt% and about 10% wt% relative to the total weight of the complete microemulsion, and more preferably between about 4.0 wt% and about 10% by weight relative to the total weight of the complete microemulsion.

The aqueous medium component D, of the microemulsion embodiments of the present invention is at least water, preferably, de-ionized water. The aqueous medium, component D may include for example in addition to water such optional stabilizers and ingredients as antioxidants, chelating agents, UV filters, and preservatives. Additional ingredients such as thickening agents, cosmetic active ingredients, moisturizers, humectants, emollients, opacifiers, pearly gloss impacting substances, pigments, colorants, dyes and antifoams may also be included in the aqueous medium of the compositional embodiments of the present invention.

The amount of aqueous medium, component D, present in the composition generally will be the remainder after subtracting the weight percentages of fragrance oil, surfactant system and preservative system from 100%. A dilution factor may also be considered such that the combination of components other than water may range from about 1.0 wt% to about 50 wt%. With the same relative amounts of components, A, B, C and optional ingredients, a concentrated aliquot may be formulated with a water content of at least about 20 wt% to 30%. This concentrated aliquot may be diluted multiple times to provide consumer acceptable formulations having water contents in the range of about 65 wt% to about 97 wt%, preferably of about 65 wt. % to about 95 wt. %, more preferably from about 65 wt. % to about 90 wt. %, especially more preferably from about 65 wt. % to about 85 wt. % and most preferably from about 65 wt% to about 80 wt%.

The surfactant system, component B of the ethanol-free microemulsion composition embodiments of the present invention includes a primary surfactant and a co-surfactant/hydrotrope. As explained above, this combination enables incorporation of a surfactant system at significantly lower concentrations that would be needed if the co-surfactant/hydrotrope were excluded. This enables minimization or elimination of olfactive responses that would otherwise occur at higher concentrations of the primary surfactant along.

The primary surfactant of surfactant system, component B, comprises any one of several families of non-ionic surfactants or combinations thereof. These family include for example polyethoxylated or polypropoxylated non-ionic surfactants. This component of the surfactant system of the embodiments of the invention includes in particular and as non-limiting examples, those belonging to the classes of mono, di or triglycerides epoxylated with polyglycols, fatty acids esterified with polyols (polyglycols) and fatty alcohols ethoxylated with polyol (polyglycols). More particularly are esters formed of a fatty acid moiety having from 15 to 50 carbons esterified with a polyol moiety such as PEG or PPG having from 6 to 80 monomeric units. Also, more particularly are polyethers formed of the fatty alcohol moiety corresponding to the fatty acid moiety ethoxylated with the foregoing polyol moiety. The fatty acid moiety may be a saturated of unsaturated form and may additionally optionally hydroxylated. Included as examples of unsaturated fatty acids are oleic, elaidic, vaccenic, linoleic, arachidonic, eicosapentenoic, erucic, docosahexenoic and castor oil precursor acids. Examples of saturated fatty acids are hydrogenated forms of the foregoing unsaturated fatty acids as well as capric, lauric, myristic, palmitic, stearic, arachidic, behenic, lignoceric and cerotic acids. Additional examples include the mono, di and tri hydroxylated forms of these saturated and unsaturated fatty acids, i.e., mono, di and tri-glycerides. Hydroxyls of the glycerin of the mono or di glyceride can be epoxylated with polyglycol or the fatty acid moiety of the triglyceride contains hydroxyls which can be epoxylated with polyglycol or a combination of the free hydroxyls of the glycerin moiety and the fatty acid moiety can be epoxylated with polyglycol. Examples of the fatty alcohols correspond to the foregoing fatty acid examples as well as the hydroxylated forms thereof. Examples of the esterifying or ethoxylating moiety include PEG or PPG moieties having from 6 to 60 monomeric units, preferably 12 to 60 monomeric units, more preferably 15 to 60 monomeric units and most preferably 25 to 55 monomeric units. Further examples include trideceth 9 (a polyoxyethylene tridecyl ether) and PEG-5 isononanoate (a polyethylene glycol esterified with a fatty acid, pelargonic acid). Additional examples include hydrogenated castor oil esterified with PEG 40 and the fatty alcohol buteth 26 ethoxylated with PPG 26 and a combination thereof.

Additional families of the primary surfactant component of the surfactant system of embodiments of the invention include sugar ester surfactants such as sucrose esters with C₈-C₂₅ fatty acids such as sucrose esters with oleic, palmitic or stearic acid and in particular, sucrose monopalmitate. Further exemplary surfactant groups include C₈-C₂₅ alkyl polyglucosides, fatty alcohol glucosides such as C₈-C₂₅ alkyl glucosides, e.g. laurylglucoside, caprylglucoside or combination thereof with cocoglucoside and glyceryl oleate, ethoxylated aliphatic C₆-C₂₅ alcohols containing 2 to 30 PEG and/or PPG units (PEG being ethylene oxide moieties and PPG being propylene oxide moieties). In particular, additional examples include C10₋₂₀ alcohol ethers with 9 PEG units, a butyl alcohol ether with 24 to 27 PEG and/or PPG oxide units (such as PPG-24 Buteth-26), a phosphoric ester of lauryl alcohol ether with 3 PEG (such as Trilaureth-4 Phosphate) and mixtures thereof. Additional families include polyethylene glycol stearyl ethers, the polyethylene glycol (n) oleyl ethers, the polyethylene glycol (n) nonylphenyl ethers, and the polysorbates.

Additional families include polyoxyethylene C₁₄₋₆₀ hydroxylesters containing 10 to 40 PEG units, such as polyoxyethylenesorbitan monooleate (for examples sorbitol monoesters with oleic, myristic, stearic or palmitic acid, e.g. sorbitol monoester with a fatty acid which are polyethoxylated and containing 10 to 40 PEG units or such as polyethoxylated castor oils triglyceride containing 10 to 40 PEG units, and C₈₋₂₅ mono- and polyglyceryl esters.

Further suitable examples of the primary surfactant component of the surfactant system, component B, include ethoxylated and/or propoxylated (C₅₋₁₂ alkyl) phenol ethers containing 5 to 20 PEG or PPG units (such as polyethylene glycol nonylphenyl ethers, polyethylene glycol octylphenyl ethers, polyethylene glycol sorbitol ether containing 3 to 30 PEG units (such as sorbitol esters with oleic, myristic, stearic, palmitic acid, sucrose esters with C₈₋₂₀ fatty acid (such as sucrose esters with oleic, palmitic or stearic acid, ethoxylated aliphatic C₆₋₂₀ alcohols containing 2 to 30 PEG units (such as ethoxylated secondary C₆₋₂₀ alcohols), C₆₋₂₀ polyglyceryl esters (such as glycerol-polyethylene glycol oxystearate, polyethylene glycol and polypropylene glycol block copolymers, ethoxylated glycol ether containing 2 to 30 EO units (such as PEG-10 stearyl ether), or polyethylene glycol mono- or -diester of aliphatic C₅₋₁₁carboxylic acids containing 2 to 10 PEG or PPG units (PEG stands for ethylene oxide and PPG stands for propylene oxide). In addition, for some embodiments of the compositions of the invention, an optional cationic and/or anionic surfactant such as a C8 to C20 fatty acid salt or a C8 to C20 alkyl ammonium salt may optionally be added in addition to the non-ionic surfactant to maintain the microemulsion dispersion. The total wt % of non-ionic surfactant and optional cationic and/or anionic surfactant falls under the wt% of the primary surfactant.

The co-surfactant/hydrotrope component of the surfactant system includes diols, triols or polyols of mono-, poly- or oligomeric nature or derivatives thereof. Linear alkane diols are preferred, and most preferable are alkane diols having from 5 to 8 carbon atoms such as 1,5-pentanediol, 1,6-hexanediol, 1,7-heptanediol, 1,8-octanediol, and mixtures thereof. Examples of monomeric diols or triols include ethyleneglycol, 1,2-propanediol, 1,3-propanediol, 1,2-butanediol, 1,3-butanediol, 1,4-butanediol, 2,3-butanediol, 2-methyl-1,3-propanediol, 1,6-hexanediol, neopentylglycol, trimethylol-propane, and glycerol. Examples of oligomeric or polymeric diols, triols or polyols include diethyleneglycol, triethyleneglycol, dipropyleneglycol, and polyethyleneglycols with varied chain lengths.

The weight percentage of primary surfactant present in the surfactant system relative to the total weight of the surfactant system ranges from a majority to substantially all of the total weight of the surfactant system, preferably from about 50 wt% to about 98 wt%, more preferably from about 60 wt% to about 95 wt%, especially more preferably from about 60 wt% to about 90 wt%, and most preferably about 65 wt% to about 80 wt% all relative to the total weight of the surfactant system. The remaining weight percentage for each of these ranges constitutes the weight percentage range for the co-surfactant/hydrotrope.

The surfactant system according to the invention is used in proportions of from about 0.1 wt% to about 25.0 wt%, preferably from about 2.0 wt% to about 23.0 wt%, more preferably about 5.0 wt% to about 20 wt% relative to the total weight of the emulsion.

The weight ratio of the fragrance oil to the primary surfactant is from 1.2:1 to 10:1, preferably from 2:1 to 6:1, more preferably from 2:1 to 5:1, and most preferably from 2:1 to 4:1.

The preservative system for the microemulsion embodiments of the invention, component C, includes at least o-cymen-5-ol (3-methyl-4-isopropyl phenol) and EDTA in combination with antibacterial and/or antimicrobial compounds such as short chain alkanoic acids, aromatic acids and alcohols, terpenoids , and similar antimicrobials. Non-limiting examples of acids, alcohols, terpenoids and other antimicrobials include acetic, citric, benzoic, valeric and adipic acids, glycols, triclorestan, triclocarban, chloroxylenol, colloidal silver, quaternary ammonium compounds, biguanide polymer and similar compounds. Specific examples of antimicrobial compounds include phenoxyethanol and a mixture of parabens. The parabens mixture includes methyl, ethyl, propyl and butyl parabens. A preferred preservative system includes the combination of EDTA, o-cymen-5-ol, phenoxyethanol, and the mixture of methyl, ethyl, propyl and butyl parabens. While the positional isomer of o-cymen-5-ol, thymol, may also be used alone or in combination with o-cymen-5-ol, the pleasant aromatic fragrance of thymol make it not preferred for at least some of the compositional embodiments of the invention such as those for which the thymol odiferous note would adulterate the scent of the perfume oil.

The weight percentages of preservative system relative to the total weight of the complete microemulsion embodiments of the invention range from about 1.0 wt% to about 5 wt%, preferably from about 1.0 wt% to about 3 wt%, more preferably about 1.0wt% to about 2 wt %, most preferably about 1.4 wt% to about 1.6 wt%.

Other ingredients that may optionally be present in the composition of the present invention may include for example antioxidants, additional chelating agents and UV filters. Additional ingredients such as thickening agents, cosmetic active ingredients, moisturizers, humectants, emollients, opacifiers, pearly gloss impacting substances, pigments, colorants, dyes and antifoams may also be optionally used in the compositional embodiments of the present invention. Such kinds of materials are well known to a person skilled in the art and do not need a more detailed description. Whenever such optional ingredients are added to the microemulsion, they can be present in a weight percentage range of from about 0.1 wt % to about 5 wt%, preferably from about 0.1 wt % to about 3 wt%, and more preferably from about 0.1 wt% to about 2.0 wt%.

The microemulsion embodiments of the invention can contain from about 5% to about 50% by weight, preferably about 10% to about 35% by weight of dispersed phase (fragrance oil phase) and from about 95% to about 50% by weight, preferably about 90% to about 65% by weight of continuous phase (aqueous phase). These values are relative to the total weight of the microemulsion embodiments.

The microemulsion embodiments of the invention show a sufficient stability during storage and shelf life to provide adequate and in many embodiments superior freedom from degradation, decomposition and oxidation, including but not limited to microbial decomposition and/or metabolism. Preferably, the microemulsion embodiments of the invention can be maintained essentially decomposition free for as long as three to four years under typical commercial conditions such as room temperature or below, above freezing, and in containers that do not permit transmission of light.

The perfume compositional embodiments according to the invention can be used for different types of consumer applications for perfumery products such as alcohol-free perfume emulsions, Eaux de toilette or Eaux de Cologne, atomizable onto the skin or hair. They may also serve to perfume surfaces of other types such as fabrics, wood or glass. In another embodiment, the compositions of the invention may even perfume the ambient air and thus be used as perfume diffusers. Another aspect of the present invention is a perfume beauty product for consumers comprising the compositional embodiments described above. Examples of such consumer products are a cologne, lotion, body splash, body mist and perfume essential. A "body splash" is a body care formulation that is applied to the body after bathing and provides a subtle hint of scent. A perfume essential is a liquid formulation of a microemulsion embodiment of the invention that is highly concentrated (low water content) and is applied as liquid drops or portion to warm spots of human skin such as the ventral side of a wrist or behind the ear.

Further consumer applications of the microemulsion embodiments of the invention are particularly suitable for the manufacture of consumer articles capable of dispensing a perfume in the surrounding space such as but not limited to automist devices, autovolatilization devices and auto air freshener devices. These consumer articles are also an object of the present invention.

Suitable consumer articles comprise a microemulsion embodiment as described above together with a suitable container and optionally a means to produce an aerosol. Non-limiting examples of such consumer article are room deodorants, or air fresheners, as well as hair or skin preparations, such as fine perfumery articles.

Further suitable consumer products are, for example, a surface cleaning product, a hygiene product, a hair care product such as shampoos, a body-care product, a cosmetic preparation, a fabric refresher, an ironing water or a wipe.

The following examples are given to illustrate the present invention. Because these examples are given for illustrative purposes only, the present invention embodied herein should not be limited thereto. Unless otherwise indicated all parts and percentages are by weight.

### Examples

### Example 1

### Preservative System

A biphasic perfume oil/ preservative system is prepared by combining:
a) one or more perfume oils such as Ludique, Blue Mood or Ethnic Ice, which are trade names for commercially available bulk perfume oils; and
b) one of the aqueous preservative systems designated as
   i. phenoxyethanol and C1 to C4 alkyl parabens;
   ii. phenoxyethanol, C1 to C4 alkyl parabens and o-cymen-5-ol;
   iii. phenoxyethanol, C1 to C4 alkyl parabens; o-cymen-5-ol and EDTA

Aliquots of the perfume oil/preservative systems a/bi, a/bii and a/biii are tested for inhibition of microbial growth and/or perfume oil degradation by microbial action. The aliquots are exposed to open environmental conditions at 40°C for one to three months.

Aliquot a/bi at all variations of concentrations of preservative system can be shown to fail microbial inhibition. Aliquot a/bii can demonstrate microbial inhibition at certain concentrations Aliquot a/biii can demonstrate microbial inhibition at all concentration variations.

The Example demonstrates that a formulation following aliquot a/biii will be suitable for ethanol-free aqueous perfumes.

### Example 2

### Surfactant System for Ethanol-free Perfume Formulation

Two surfactant formulations were prepared by mixing the following components:
i. Trideceth - 9 and PEG - 5 isononanoate
ii. PEG-40 hydrogenated castor oil, PPG-26-buteth 26

Each of these surfactant systems were combined with a co-surfactant/hydrotrope pentylene glycol. Ratios of surfactant formulation to co-surfactant/hydrotrope by weight tested were: 1:1 and 2.5:1 and 3:1 and 4:1. It can be discovered that the 2.5:1 and 3:1 ratios are appropriate.

### Example 3

### Complete Ethanol-free Perfume Formulations

An ethanol-free, aqueous microemulsion of perfume oil is prepared by combining the perfume oil preservative formulation of Example 1a/biii with a 2.5:1 surfactant system composed of:
a) PEG-40 hydrogenated castor oil, PPG-26-buteth 26 as primary surfactant I; or Trideceth - 9 and PEG - 5 isononanoate as primary surfactant II
b) Pentylene glycol as a co-surfactant/hydrotrope
c) Water

The weight percent concentrations of the components are 15 wt% perfume oil; 1.45 wt% preservative system; 12 wt% primary surfactant; 5.0 wt% co-surfactant/hydrotrope and a remainder of deionized water (66 wt%).

The primary surfactant and co-surfactant/hydrotrope are combined with water by gentle stirring to form a uniphase liquid. Next the components of the preservative system are slowly added to the stirring uniphase liquid. Following complete addition of the preservative system, the perfume oil is added dropwise while stirring the aqueous medium. A microemulsion embodiment of the invention is produced.

It can be discovered that the microemulsion composition with surfactant system a) above produced a stable microemulsion that can be sustained for as long as three years. It can be discovered that the microemulsion composition with surfactant system b) above produced a stable microemulsion that can be sustained for as long as one year.

With both microemulsion compositions, microbial growth can be essentially inhibited for as long as three years.

### Statements of the Invention

The following statements of the invention disclose embodiments of the invention.

### Statements:

1. A composition comprising an ethanol-free, aqueous microemulsion of at least a preservative system, a surfactant system, fragrance oil and water.
2. A composition according to statement 1 wherein the preservative system is a combination of o-cymen-5-ol (3-methyl-4-isopropyl phenol), EDTA, phenoxyethanol and at least one parabens.
3. A composition according to statement 2 wherein the at least one parabens is a mixture of parabens.
4. A composition according to any one of statements 1-3 or any combination thereof wherein the surfactant system comprises a primary surfactant and a co-surfactant/hydrotrope.
5. A composition according to statement 4 wherein the primary surfactant is a non-ionic surfactant having a polyethylene glycol or polypropylene glycol tail and a head of a saturated or unsaturated, mono, di or triglyceride optionally containing hydroxyl groups.
6. A composition according to statement 6 wherein the primary surfactant is at least one of PEG-40 hydrogenated castor oil, PPG-26 Buteth-26, or any combination thereof.
7. A composition according to any one of statements 5 and 6 or any combination thereof wherein the co-surfactant/hydrotrope is a C5 to C10 alkyl diol or triol or a combination of two or more diols or triols.
8. A composition according to statement 7 wherein the co-surfactant/hydrotrope is any one of pentylene glycol, 1,6 hexanediol, caprylyl glycol or any combination thereof.
9. A composition according to any one of statements 7 and 8 wherein the primary surfactant is PEG-40 hydrogenated castor oil, PPG-26-Buteth-26 or a combination thereof.
10. A composition according to any one of statements 8 and 9 wherein the co-surfactant/hydrotrope is pentylene glycol.
11. A composition according to statement 10 wherein the surfactant is PEG-40 hydrogenated castor oil and PPG-26-Buteth-26 and the co-surfactant/hydrotrope is pentylene glycol.
12. A composition according to any one of statements 1-11 or any combination thereof wherein the weight percentages relative to the total weight of the composition are: for the perfume oil disperse phase - about 2.0 wt% to about 15 wt%, preferably about 2.0 wt% to about 10 wt%; for the surfactant system - about 0.1 wt% to about 25.0 wt%, preferably about 2.0 wt% to about 15 wt%; for the preservative system - about 1.0 wt% to about 5 wt%, preferably about 1.2 wt% to about 2.5 wt%; and a remainder of water.
13. A composition according to any one of statements 1-12 or any combination thereof wherein the microemulsion comprises microdroplets of perfume oil in an aqueous continuous phase, the microdroplets having a diameter size of about 10 nanometers to 100 nanometers.
14. A composition according to any one of statements 1-13 or any combination thereof further comprising a UV inhibitor.
15. A composition according to statement 14 wherein the UV inhibitor is any one or more of benzophenone, ethylhexyl methoxycinnamate, diethylamino hydroxybenzoyl hexyl benzoate, ethylhexyl triazone oxybenzone, octinoxate, octocrylene, PEG-25 PABA, sulisobenzone.
16. A composition comprising an ethanol-free, aqueous microemulsion comprising a primary surfactant of a PEG-40 hydrogenated castor oil and PPG-26-Buteth-26, a co-surfactant/hydrotrope ofpentylene glycol, a preservative of phenoxyethanol, o-cymen-5-ol, EDTA, methyl paraben, ethyl paraben, propyl paraben and butyl paraben, a fragrance oil and water.
17. A composition according to any one of statements 1 - 16 or any combination thereof wherein the weight percentages relative to the total weight of the composition are about 4.0 wt% to about 8.0 wt% fragrance oil; about 0.01 wt% to about 18 wt% primary surfactant; about 0.1 wt% to about 5.0 wt% co-surfactant;, about 1.0 wt% to about 3.0 wt% preservative; and a remainder of water.
18. A composition according to statement 17 wherein the fragrance oil is in the form of microdroplets in an aqueous microemulsion and the microdroplets have a diameter size of 10 nanometers to 100 nanometers.
19. A composition according to any one of statements 1-16 or any combination thereof which is essentially free of microbial growth for a period of at least three months at 40°C and 75 % humidity.
20. A composition according to any one of statements 1-18 or any combination thereof which is essentially free of microbial growth for a period of at least three years at ambient temperature and humidity.
21. A composition according to any one of statements 1-18 or any combination thereof which exhibits microemulsion stability for at least three years at ambient temperature and humidity.
22. A composition according to statement 21 which exhibits microemulsion stability for at least 12 weeks at 40°C and 75% humidity.
23. A composition according to any one of statements 1-22 exhibiting visible light transmission of clear to translucent.
24. A method for producing an ethanol-free, aqueous microemulsion of a fragrance oil, a preservative system, a surfactant system and water, comprising
   a. Combining fragrance oil, water, the surfactant system and the preservative system to form a mixture
   b. Homogenizing the mixture to form microdroplets of the fragrance oil wherein the microdroplets have an average diameter of 10 nanometers to 100 nanometers.
25. A method according to statement 24 wherein the microdroplets are stabilized by the surfactant system and the surfactant system comprises a primary surfactant and a co-surfactant/hydrotrope.
26. A method according to statement 24 wherein the primary surfactant is at least one of PEG-40 hydrogenated castor oil, PPG-26 Buteth-26, or any combination thereof and the co-surfactant/hydrotrope is any one of, pentylene glycol, 1,6 hexanediol, caprylyl glycol or any combination thereof.
27. A method according to statement 26 wherein the primary surfactant is a combination of PEG-40 hydrogenated castor oil and PPG-26 Buteth-26; and the co-surfactant/hydrotrope is pentylene glycol.
28. A method according to any one of statements 24-27 wherein the preservative system is a combination of o-cymen-5-ol (3-methyl-4-isopropyl phenol), EDTA, phenoxyethanol and a mixture ofparabens.
29. Use of a composition according to any one of statements 1-23 as a perfume by application of the composition to skin of a human.
30. Use of a composition according to statement 30 to provide a clean, soft feel to skin to which the composition is applied.
31. Use of a composition according to statement 30 to provide a substantially long lasting pleasant fragrance for the human to which the composition is applied.
32. Use of a composition according to statement 29 wherein the composition exhibits microbial and microemulsion stability for at least 3 years of shelf life.
33. A composition according to any one of statements 1-23 wherein the microemulsion of microdroplets of perfume oil is stabilized by the combination of a primary surfactant and a co-surfactant/hydrotrope.
34. A composition according to any one of statements 1-23 and 31 wherein the perfume oil is a natural or synthetic substance of mixture thereof that presents olfactive properties.
35. A composition according to any one or more of statements 1-23, 33, 34 further comprising a minor amount of ethanol.
36. A composition according to statement 35 wherein the minor amount of ethanol is less than 1 wt% relative to the total weight of the composition.
37. A composition according to statement 36 wherein the minor amount of ethanol is less than 0.5 wt% relative to the total weight of the composition.
38. Use of a composition according to any one or more of statements 29-32 wherein the composition further comprises a minor amount of ethanol.
39. Use of a composition according to statement 38 wherein the amount of ethanol is less than 1 wt% relative to the total weight of the composition.
40. Use of a composition according to statement 38 wherein the amount of ethanol is less than 0.5 wt% relative to the total weight of the composition.
41. A composition according to statement 3 wherein the mixture of parabens comprises methyl, ethyl, propyl and butyl parabens.

### General Statements

The inventions, examples, biological assays and results described and claimed herein have may attributes and embodiments include, but not limited to, those set forth or described or referenced in this application.

The term "may" in the context of this application means "is permitted to" or "is able to" and is a synonym for the term "can." The term "may" as used herein does not mean possibility or chance.

All patents, publications, scientific articles, web sites and other documents and ministerial references or mentioned herein are indicative of the levels of skill of those skilled in the art to which the invention pertains, and each such referenced document and material is hereby incorporated by reference to the same extent as if it had been incorporated verbatim and set forth in its entirety herein. The right is reserved to physically incorporate into this specification any and all materials and information from any such patent, publication, scientific article, web site, electronically available information, text book or other referenced material or document. The written description of this patent application includes all claims. All claims including all original claims are hereby incorporated by reference in their entirety into the written description portion of the specification and the right is reserved to physically incorporate into the written description or any other portion of the application any and all such claims. Thus, for example, under no circumstances may the patent be interpreted as allegedly not providing a written description for a claim on the assertion that the precise wording of the claim is not set forth in haec verba in written description portion of the patent.

All features disclosed in this specification may be combined in any order and in any combination with any of the embodiments of the disclosed perfume composition.

While the invention has been described in conjunction with the detailed description thereof, the foregoing description is intended to illustrate and not limit the scope of the invention, which is defined by the scope of the appended claims. Thus, from the foregoing, it will be appreciated that, although specific nonlimiting embodiments of the invention have been described herein for the purpose of illustration, various modifications may be made without deviating from the spirit and scope of the invention. Other aspects, advantages, and modifications are within the scope of the following claims and the present invention is not limited except as by the appended claims.

The specific methods and compositions described herein are representative of preferred nonlimiting embodiments and are exemplary and not intended as limitations on the scope of the invention. Other objects, aspects, and embodiments will occur to those skilled in the art upon consideration of this specification, and are encompassed within the spirit of the invention as defined by the scope of the claims. It will be readily apparent to one skilled in the art that varying substitutions and modifications may be made to the invention disclosed herein without departing from the scope and spirit of the invention. The invention illustratively described herein suitably may be practiced in the absence of any element or elements, or limitation or limitations, which is not specifically disclosed herein as essential. Thus, for example, in each instance herein, in nonlimiting embodiments or examples of the present invention, the terms "comprising", "including", "containing", etc. are to be read expansively and without limitation. The methods and processes illustratively described herein suitably may be practiced in differing orders of steps, and that they are not necessarily restricted to the orders of steps indicated herein or in the claims.

The terms and expressions that have been employed are used as terms of description and not of limitation, and there is no intent in the use of such terms and expressions to exclude any equivalent of the features shown and described or portions thereof, but it is recognized that various modifications are possible within the scope of the invention as claimed. Thus, it will be understood that although the present invention has been specifically disclosed by various nonlimiting embodiments and/or preferred nonlimiting embodiments and optional features, any and all modifications and variations of the concepts herein disclosed that may be resorted to by those skilled in the art are considered to be within the scope of this invention as defined by the appended claims.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

It is also to be understood that as used herein and in the appended claims, the singular forms "a," "an," and "the" include plural reference unless the context clearly dictates otherwise, for example, the term "X and/or Y" means "X" or "Y" or both "X" and "Y", and the letter "s" following a noun designates both the plural and singular forms of that noun. In addition, where features or aspects of the invention are described in terms of Markush groups, it is intended, and those skilled in the art will recognize, that the invention embraces and is also thereby described in terms of any individual member and any subgroup of members of the Markush group, and the right is reserved to revise the application or claims to refer specifically to any individual member or any subgroup of members of the Markush group. A Markush group is indicated by any one of a number of phrases including "selected from the group consisting of", a series of atoms, groups or molecules ending with the penultimate term "or" and a series of atoms groups or molecules introduced by a phrase such as "selected from" or "chosen from."

## Claims

1. A composition comprising an ethanol-free, aqueous microemulsion of at least a preservative system, a surfactant system, fragrance oil and water wherein the preservative system is a combination of o-cymen-5-ol, EDTA, phenoxyethanol and at least one parabens and the surfactant system is a mixture of a primary surfactant and a co-surfactant/hydrotrope.

2. A composition according to claim 1 wherein the at least one parabens comprises a mixture of parabens.

3. A composition according to claim 2 wherein the mixture comprises methyl, ethyl, propyl and butyl parabens.

4. A composition according to claim 1, 2 or 3 or any combination thereof wherein the primary surfactant is PEG-40 hydrogenated castor oil, PPG-26-Buteth-26 or a combination thereof.

5. A composition according to claim 4 wherein the co-surfactant/hydrotrope is pentylene glycol.

6. A composition according to claim 1 wherein the weight percentages relative to the total weight of the composition are: for the perfume oil disperse phase - about 2.0 wt% to about 15 wt%, preferably about 2.0 wt% to about 10 wt%; for the surfactant system - about 0.1 wt% to about 25.0 wt%, preferably about 2.0 wt% to about 15 wt%; for the preservative system - about 1.0 wt% to about 5 wt%, preferably about 1.2 wt% to about 2.5 wt%; and a remainder of water.

7. A composition according to claim 1 further comprising a UV inhibitor.

8. A composition according to claim 7 wherein the UV inhibitor is any one or more of benzophenone, ethylhexyl methoxycinnamate, diethylamino hydroxybenzoyl hexyl benzoate, ethylhexyl triazone oxybenzone, octinoxate, octocrylene, PEG-25 PABA, sulisobenzone.

9. A composition according to claim 1 which is essentially free of microbial growth for a period of at least 12 weeks at 40°C and 75 % humidity.

10. A composition according to claim 1 which is clear to translucent to visible light.

11. Use of a composition according to claim 1 as a perfume by application of the composition to skin of a human.

12. Use of a composition according to claim 11 wherein the composition exhibits microbial and microemulsion stability for at least 3 years of shelf life by the presence of at least 2 wt% of the preservative system relative to the total weight of the composition.

13. A composition according to claim 1 further comprising no more than 1 wt% of ethanol relative to the total weight of the composition.

14. A composition according to claim 1 that is completely free of ethanol.
